Europäisches Patentamt

European Patent Office

Office européen des brevets

(11) Publication number: **0 207 475 B1**

## (12) EUROPEAN PATENT SPECIFICATION

(45) Date of publication of patent specification: **23.09.92**  (51) Int. Cl.⁵: **C12P 7/64**, C12N 1/38

(21) Application number: **86108818.5**

(22) Date of filing: **28.06.86**

(54) Process for secretive fermentation of lipids by fungi or algae.

(30) Priority: **01.07.85 JP 144371/85**

(43) Date of publication of application:
**07.01.87 Bulletin 87/02**

(45) Publication of the grant of the patent:
**23.09.92 Bulletin 92/39**

(84) Designated Contracting States:
**DE GB**

(56) References cited:
**CH-A- 494 278**
**GB-A- 2 091 285**
**GB-A- 2 091 286**
**US-A- 2 697 061**
**US-A- 2 697 062**

**AGR. BIOL. CHEM., vol. 40, no. 4, 1976, pages 719-723; K. YAMAGUCHI et al.: "Studies on the fatty acid compositions of lipid from Mycotorula japonica"**

**BIOTECHNOLOGY AND BIOENGINEERING, vol. 19, 1977, pages 781-790; B. METZ et al.: "The growth of molds in the form of pellets- a literature review"**

**JOURNAL OF GENERAL MICROBIOLOGY, vol. 68, 1971, pages 245-247, GB; I.H.C. GAL-LAGHER: "Occurrence of waxes in ac-inetobacter"**

**CHEMICAL ABSTRACTS, vol. 107, no. 1, July 1987, page 543, abstract no. 5785x, Colum-bus, Ohio, US; & JP-A-62 06 694 (KANEGAFUCHI CHEMICAL INDUSTRY CO., LTD) 13-01-1987**

(73) Proprietor: **KANEGAFUCHI KAGAKU KOGYO KABUSHIKI KAISHA**
**2-4 Nakanoshima 3-chome**
**Kita-ku Osaka-shi Osaka-fu 530(JP)**

(72) Inventor: **Fukuda, Hideki**
**16-25, Nishihata 1-chome**
**Takasago-shi Hyogo-ken(JP)**

(74) Representative: **Türk, Gille, Hrabal, Leifert**
**Brucknerstrasse 20**
**W-4000 Düsseldorf 13(DE)**

EP 0 207 475 B1

**Description**

the present invention relates to a process for preparing secreted lipids, optionally in combination with non-secreted lipids, by employing fungi or algae capable of biosynthesizing lipids which comprises conducting the reaction in a reaction system wherein surfactants are present.

Not only lipids themselves produced by microorganism cell can be used as starting materials for oils and fats but also useful substances such as varieties of sterols and higher fatty acids, which are obtained by purification of the lipids, can also be utilized for a wide application in the fields of food, drug and general industry.

Therefore, hitherto energetical studies have been made to improve the productivity of lipids or to produce lipids in a desirable composition.

In recent years, this technical field is increasingly drawing public attention since it has now been made clear that, among unsaturated fatty acids recovered from the above-mentioned lipids, arachidonic acid or $\gamma$-linolenic acid has various physiological activities.

For preparing such lipids, a process for preparing cacao butter-like oils and fats (JP-A-75292/1985), a process for preparing arachidonic acid (JP-B-64482/1977, "Biotechnology and Bioengineering", Vol. 25, 1057 (1983)), and a process for preparing $\gamma$-linolenic acid (JP-B-22280/1972 and 22199/1983) are already known.

Furthermore, from US-A-2697061, GB-A-2091285 and "Agr. Biol. Chem." (1976), 40(4), p. 719 - 723, the production and isolation of lipids synthesized by fungi in the presence of surfactants in the culture medium are known.

However, these processes have disadvantages, i.e. the amount of lipids accumulated in various microorganism cells is limited and thus it is impossible to produce lipids in a sufficient amount. Further, since most of lipids are accumulated in the cells, complex extraction procedures such as crushing or acid treatment of the cells are required in order to recover the lipids. When such extraction procedure is carried out, a purification process becomes extremely complicated due to a large amount of impurities extracted, which has been an obstacle for an industrialization.

Therefore, the object of the present invention is to find a novel process for preparing lipids wherein lipids can be prepared in high yield and can be easily recovered.

As the result of continuous efforts, it has been found that the lipids accumulated in the cell can be secreted in a large amount and the lipids can be easily recovered in high yield, when immobilized microorganism particle of the fungi or algae capable of biosynthesizing lipids is employed in the reaction system wherein surfactants are present. In this manner, the lipids can more efficiently be prepared in high yield since the concentration of the microorganisms in the reaction vessel is maintained at high concentration.

Although a process for preparing glutathione in yeast cells is already known (JP-A-86691/1979 as the process wherein useful substances prepared in the cell are secreted into the reaction solution, this process is distinguished from the process of the present invention in the kind of microorganisms and of substances accumulated. Thus, the process for preparing lipids wherein produced lipids are efficiently secreted into the reaction solution has never been known.

In accordance with the present invention there is provided a process for preparing secreted lipids optionally in combination with non-secreted lipids by employing fungi or algae capable of biosynthesizing lipids which comprises conducting the reaction in a reaction system wherein surfactants are present, which is characterized in that immobilized microorganism particle of fungi or algae is employed.

According to the process of the present invention, secreted lipids can be accumulated in the reaction solution in a large amount. Although the mechanism that the lipids are smoothly secreted out of the cells is not clearly known, it is presumed that the surfactants act on the surface of the microorganism cells and improve the permeability of the cell membranes without adversely affecting the growth of the microorganisms if the surfactants is present in the reaction solution under proper reaction condition. From the thus obtained lipids in the reaction solution, i.e. oils and fats, various sterols, and various higher fatty acids can be easily recovered in high yield by the usual extraction and purification processes.

Any fungi and algae can be employed in the present invention as far as the microorganism is capable of biosynthesizing lipids. Useful examples of the microorganisms are those belonging to the genus of (1) Aspergillus, (2) Fusarium, (3) Penicillium, (4) Porphyridium, (5) Mucor, (6) Rhizopus, (7) Absidia, (8) Actinomucor, (9) Choanephora, (10) Cunninghamella, (11) Phycomyces, (12) Rhizomucor, (13) Helicostylum, (14) Paecilomyces, (15) Syncephalastrum, (16) Circinella, (17) Gongronella, (18) Backusella, (19) Pythium, (20) Fennellomyces, (21) Mortierella, (22) Spirulina, (23) Cladosporium, (24) Trichoderma, (25) Pellicularia, (26) Gliocladium, (27) Humicola, (28) Claviceps, (29) and Chlorella,. Particularly, the above microorganisms

2

(1) to (6) are preferably employed for the production of arachidonic acid while the microorganisms (5) to (22) are preferably employed for the production of γ-linolenic acid in view point of the yield.

Typical preferred examples of the strain belonging to the above each genus are, for instance, Aspergillus candidis IFO 4309, Fusarium oxysporum IFO 5942, Penicillium spinulosum IFO 5793, Porphyridium cruentum, Mucor ambiguus IFO 6742, Rhizopus oryzae IFO 5418, Rhizopus stolonifer IFO 4781, Absidia coerulea IFO 4011, Actinomucor repens HUT 1049, Choanephora circinans IFO 5991, Cunninghamella echinuleta var. elegans IFO 4441, Phycomyces nitens IFO 5694, Phizomucor miehei IFO 9740, Helicostylum nigricans IFO 8091, Paecilomyces varioti HUT 4028, Syncephalastrum nigricans HUT 1229, Circinella rigida IFO 6411, Gongronella butleri IFO 8080, Backusella circina IFO 9231, Pythium debaryanum IFO 5919, Fennellomyces linderi IFO 6409, Martirella isabellina IFO 7824, Spirulina platensis, Cladosporium herbarum IFO 6348, Trichoderma viride IFO 9066, Pellicularia filamentosa IFO 6476, Gliocladium virens IFO 9169, Humicola grisea IFO 4868, Claviceps purpurea IFO 5782, and Chlorella pyrenoidosa,.

The culture condition of such microorganisms is not particularly limited as far as the microorganisms can grow. For the fungi, the medium containing carbohydrates such as glucose, molasses and sucrose, ethanol, acetic acid or hydrocarbons such as n-alkane, n-decane, hexadecane and heptadecane as a carbon source, to which other necessary nutrient sources are added, is generally employed. Conditions such as temperature, pH and reaction time can be adjusted to those suitable for each microorganism. Also for the algae, the commonly known inorganic nutrient source is employed as the basic medium and the conditions such as illumination intensity, temperature, pH and reaction time can be properly adjusted for each microorganism.

The surfactants present in the reaction system of the present invention may be any of cationic surfactants, anionic surfactants, nonionic surfactants or amphoteric surfactants. Among them, anionic surfactants and nonionic surfactants are particularly effective.

As the anionic surfactants, compounds having properties of anionic surfactants, e.g. (1) carboxylate type high molecular surface active agent, (2) polyoxyethylene alkyl ether sulfate, (3) polyoxyethylene alkyl aryl ether sulfate, (4) dialkyl sulfosuccinate, (5) alkylbenzene sulfonate, (6) alkyl sulfate, (7) fatty acid salt, (8) alkyl phosphate, (9) alkyl naphthalene sulfonate, (10) aromatic sulfonic acid formaldehyde condensate, and (11) naphthalene sulfonic acid formaldehyde condensate, can be used. Among them, the compounds (1) to (9) are especially effective. As the typical example of commercially available product, the compound (1) includes Demol EP (made by Kao Corporation), Poiz 540 (made by Kao Corporation), Eleminol MBN-1 (made by SANYO CHEMICAL INDUSTRIES, LTD.), Eleminol MBN-2 (made by SANYO CHEMICAL INDUSTRIES, LTD.), Sanspal PS-8 (made by SANYO CHEMICAL INDUSTRIES, LTD. ), and Polystar OM (made by Nippon Oil and Fats Co., Ltd.); the compound (2) includes Emal 20 C (made by Kao Corporation) and the like; the compound (3) includes Sannol NES (made by LION CORPORATION), and Emal NC (made by Kao Corporation); and the compound (4) includes Pelex OT-P (made by Kao Corporation), and Rapisol B-30 (made by Nippon Oil and Fats Co., Ltd.).

As the nonionic surfactants, compounds having properties of nonionic surfactants, e.g. (1) polyoxyethylene sorbitan fatty acid ester, (2) polyoxyethylene polypropylene blockpolymer, (3) sorbitan fatty acid ester, (4) polyethylene glycol fatty acid ester, (5) polyoxyethylene alkylether, (6) polyoxyethylene alkylphenylether, (7) fatty acid monoglyceride, and (8) polyoxyethylene alkylamine can be used. Among them, the compounds (1) to (4) are especially effective. As the typical example of the commercially available product, the compound (1) includes, for example, Nissan nonion LT-221 (made by Nippon Oil and Fats Co., Ltd.), Rheodol TW-L120 (made by Kao Corporation), and Tween 20 (made by Nakarai Chemicals, Ltd.) for polyoxyethylene sorbitan monolaurate; Rheodol TW-P120 (made by Kao Corporation), and Tween 40 (made by Nakarai Chemicals, Ltd.) for polyoxyethylene sorbitan monopalmitate; and Rheodol TW-S120 (made by Kao Corporation) for polyoxyethylene sorbitan monostearate; Nissan nonion OT-211 (made by Nippon Oil and Fats Co., Ltd.), Rheodol TW-0120 (made by Kao Corporation), and Tween 80 (made by Nakarai Chemicals, Ltd.) for polyoxyethylene sorbitan monooleate; and Rheodol TW-0320 (made by Kao Corporation) for polyoxyethylene sorbitan trioleate. The compound (2) includes Plonon 201, and Plonon 102 (made by Nippon Oil and Fats Co., Ltd.); the compound (3) includes Nissan nonion OP-85R (made by Nippon Oil and Fats Co., Ltd.), Rheodol SP-030 (made by Kao Corporation), and Emasol O-30(F) (made by Kao Corporation); the compound (4) includes Emanon 4110 (made by Kao Corporation).

A mixture of the anionic surfactants and the nonionic surfactants in a proper ratio can also be employed. The surfactants may be added to the reaction system either at an early stage of the reaction, or during the reaction or at a late stage of the reaction to effectively secrete the lipids. If these surfactants induce severe foaming, a suitable amount of the commonly used antifoaming agent may be added.

In order to allow the lipids to be accumulated in the reaction solution effectively, an amount of the surfactants present in the reaction system, ranges in case of the nonionic surfactants, from 0.005 to 5 % by weight, preferably from 0.01 to 1 % by weight since a concentration of not more than 0.05 % by weight cannot provide effective action to the cell membrane and a concentration of not less than 5 % by weight has an unfavorable effect on the growth of the microorganism. In case of the nonionic surfactants, an amount of the surfactants present in the reaction system ranges from 0.05 to 8 % by weight, preferably from 0.3 to 3 % by weight by the same reason.

Although the microorganism may be cultured by the conventional culture such as batch culture, semibatch culture or continuous culture in the usual suspension system, the culture is more efficiently conducted if the microorganism is immobilized as the immobilized microorganism.

For immobilization, either entrapping method using a gelling agent or physical adsorption method using a microorganism supporting material may be employed. As the gelling agent, commonly known substances such as polyacrylamide, alginic acid, carrageenan, collagen and urethane polymer. As the microorganism supporting material, any porous material can be employed which enables immobilization through sticky property of the fungi or the algae. For example, polymer foam material such as polyolefine polymer such as polyethylene or polypropylene, diene polymer such as butadiene or isoprene, polyurethane, vinyl polymer such as polyvinyl chloride, polyacrylamide or polystyrene, condensation polymer such as polyether, polyester, polycarbonate or nylon, silicone or fluorine-contained resin; inorganic material such as ceramic, glass, active carbon, pumice or metal can be employed. In order to effectively immobilize the microorganism on these microorganism supporting material, the material may preferably be a porous material having a porosity of 70 to 99 % and a pore number of 2 to 50 per linear length of the material, or a processed metal material having a porosity of 70 to 99 %.

Generally, the microorganisms as mentioned above show a pellet-like or mycelioid appearance as they grow. When the material having the above-mentioned properties is employed, the microorganism is adsorbed and immobilized during the growth of the microorganism through sticky property of the microorganism itself or entrapping by the supporting material. Various supporting materials can suitably be applied depending on a kind of the microorganism, the reaction condition or the like. The material processed into a form such as, for instance, globe, block, ring, tube or sheet may be employed. Although a size of the material may be varied depending on a kind of the microorganism, the reaction condition, a kind of the reaction vessel or the like, generally the material in the globular form may have a diameter of 1 to 100 mm and the material in the form of block may have a side length of 1 to 100 mm.

The immobilization of the microorganism to the above-mentioned supporting material can easily be conducted by a known culture such as batch culture, semibatch culture or continuous culture. For example, the empty supporting material, which is previously sterilized by steam sterilization and the like, and the microorganism such as, for instance, Mucor ambiguus IFO 6742 are subjected to an aerotic culture under favorable reaction conditions such as a basic medium of glucose, initial pH of 6.0 and a temperature of 30°C. Under these condition, the microorganism is adsorbed to the supporting material after about 100 hours to give the immobilized microorganism suitable for the reaction. Similarly, the other fungi or algae are easily immobilized in the same manner under the favorable reaction condition. The thus obtained immobilized microorganism can conduct the reaction successively in the same reaction vessel in the presence of the surfactants by batch culture, semibatch culture, continuous culture or the like. In case of batch culture or semibatch culture, the medium is replaced with the fresh medium in order to continue the reaction. Such replacing procedure may be repeated to improve the efficiency. In case of continuous culture, the fresh medium is continuously introduced after immobilization, into the reaction vessel by means of the pump whereas the reaction solution is continuously drained from one end of the reaction vessel at the same rate as the introduction of the fresh medium.

In the present invention, the aeration is conducted by air, oxygen or mixed gas thereof. Although the reaction vessel employed in the present invention may be any bubbling type with stirrer or without stirrer, usually the vessel without stirrer is preferable in view point of the operatability and the cost when the immobilized microorganism is employed.

The thus obtained lipids in the reaction solution can be recovered by the conventional purification procedure such as solvent extraction. Further, various higher fatty acids or sterols can also be obtained in high purity by known method for separation and purification such as chromatography. The lipids remained in the cells can also be recovered after treatment such as homogenization, and the various useful substances are obtained.

According to the process of the present invention, the lipids can be obtained in much higher yield than the conventional process wherein surfactants is not present. Further, the process of the present invention can simplify the purification process and produce the lipids in high purity and in high yield, which is advantageously suitable for producing lipids in an industrial process.

The present invention is more particularly explained by the following Examples.

In the following Examples, the lipids prepared in the reaction solution was analyzed by a conventional gravimetric method after solvent extraction with chloroform. After solvent extraction, hydrolysis and methylesterification, various higher fatty acids were analyzed by a gas chromatography. The lipids remained in the cells were also analyzed in the same manner as in the case of the lipids prepared in the reaction solution after homogenization of the cells.

Example 1

Various fungi were cultured by a shaking culture on the (A) medium having the composition: 4 % of glucose, 0.3 % of $KH_2PO_4$, 0.2 % of $(NH_4)_2SO_4$, 0.1 % of $(NH_2)_2CO$, 0.05 % of $MgSO_4 \cdot 7H_2O$, 0.001 % of $FeSO_4 \cdot 7H_2O$, 0.001 % of $CaC\ell_2 \cdot 2H_2O$, 0.00002 % of $CuSO_4 \cdot 5H_2O$, 0.0001 % of $ZnSO_4 \cdot 7H_2O$, 0.0001 % of $MnC\ell_2 \cdot 4H_2O$, 0.01 % of $NaC\ell$, 0.04 % of yeast extract, 0.04 % of malt extract and 0.02 % of polypeptone, or on the (B) medium having the composition: 3 % of glucose, 0.3 % of $KH_2PO_4$, 0.3 % of $NH_4NO_3$, 0.03 % of $MgSO_4 \cdot 7H_2O$, 0.02 % of yeast extract, 0.02 % of malt extract, 0.001 % of $FeSO_4 \cdot 7H_2O$, 0.00012 % of $CaC\ell_2 \cdot 2H_2O$, 0.00002 % of $CuSO_4 \cdot 5H_2O$ and 0.0001 % of $ZnSO_4 \cdot 7H_2O$, with the initial pH of 6.0 at 30°C for about 1 week to prepare seed cells. A 500 ml shaker flask was charged with the same medium as that employed in preparing seed cells, i.e. 100 ml of the (A) medium or 200 ml of the (B) medium, together with the above seed cells, and then the shaking culture was conducted in the presence of surfactants with the initial pH of 6.0 at 30°C for about 1 week to produce lipids in the reaction solution.

Spirulina platensis and Chlorella pyrenoidosa were inoculated on 500 ml of the (C) medium having the composition: 0.5 % of $NaC\ell$, 0.5 % of $KC\ell$, 0.8 % of $Na_2CO_3$, 0.1 % of $KNO_3$, 0.01 % of $MgSO_4 \cdot 7H_2O$, 0.02 % of sodium citrate, 1 ml/$\ell$ of $A_5$ solution, 1 ml/$\ell$ of iron (II) sulfate solution, 100 ml/$\ell$ of soil decotion and 40 $\mu$g/$\ell$ of vitamin $B_{12}$, and the reaction was conducted in the presence of surfactants at 30°C at pH 6.0 at illumination by fluorescent lamp of about 5000 lux for about 6 days. Porphyridium cruentum was inoculated on 100 ml of the (D) medium having the composition: 2.7 % of $NaC\ell$, 0.1 % of $KNO_3$, 0.007 % of $KH_2PO_4$, 0.004 % of $NaHCO_3$, 0.66 % of $MgSO_4 \cdot 7H_2O$, 0.56 % of $MgC\ell_2 \cdot 6H_2O$, 0.15 % of $CaC\ell_2 \cdot 2H_2O$, $4 \times 10^{-6}$ % of $ZnC\ell$, $6 \times 10^{-5}$ % of $H_3BO_4$, $1.5 \times 10^{-6}$ % of $CoC\ell_2 \cdot 6H_2O$, $4 \times 10^{-6}$ % of $CuC\ell_2 \cdot 2H_2O$, $4 \times 10^{-5}$ % of $MnC\ell_2 \cdot 4H_2O$, $3.7 \times 10^{-5}$ % of Mo, 1 ml/$\ell$ of $FeC\ell_2 \cdot 4H_2O$ (240 mg/100 ml 0.05 M $Na_2EDTA$) and 20 ml/$\ell$ of 1 M Tris-HC$\ell$ solution (pH 7.6), and the reaction was conducted in the presence of surfactants at 20°C at illumination by fluorescent lamp of about 8000 lux for about 10 days. As the surfactants, nonionic surfactants Rheodol TW-0320 was added at the concentration of 1.0 % by weight in the reaction solution. The result was compared with the control wherein the reaction was conducted in the absence of the surfactants.

The results obtained from the reaction of Mucor ambiguus IFO 6742 on the (A) medium and from the reaction of Fusarium oxysporum IFO 5942 on the (B) medium are shown in Table 1 and Table 2. Table 1 shows each amount of lipids secreted in the reaction solution and those remained in the microorganism cells. Table 2 shows the fatty acid composition of the lipids in the reaction solution prepared by the reaction in the presence of surfactants.

Table 3 shows a ratio between the amount of the lipid, arachidonic acid or $\gamma$-linolenic acid, in the reaction solution and a total amount of the produced lipids.

In these Tables, figures in parenthesis show the results of the control.

Arachidonic acid and $\gamma$-linolenic acid obtained as above were separated and purified by the conventional technique such as chromatography to produce products in high purity of not less than 97 %.

In Table 3, the results show the ratio between an amount of arachidonic acid in the reaction solution and a total amount of arachidonic acid by employing the (B) medium with respect to the microorganism in column 1; the ratio between an amount of arachidonic acid in the reaction solution and a total amount of arachidonic acid by employing the (D) medium with respect to the microorganism in column 2; the ratio between an amount of $\gamma$-linolenic acid in the reaction solution and a total amount of $\gamma$-linolenic acid by employing the (A) medium with respect to the microorganism in column 3; the ratio between an amount of $\gamma$-linolenic acid in the reaction solution and a total amount of $\gamma$-linolenic acid by employing (C) medium with respect to the microorganism in column 4; the ratio between an amount of the lipid in the reaction solution and a total amount of the lipid by employing the (C) medium with respect to the microorganism in column 5, respectively.

## Table 1

| | Strain | | | |
|---|---|---|---|---|
| | Mucor ambiguus IFO 6742 | | Fusarium oxysporum IFO 5942 | |
| (1) Dry weight of the cell (mg) | 670 | (679) | 965 | (880) |
| (2) Amount of lipid in the reaction solution (mg) | 62 | (5.2) | 116 | (6.9) |
| (3) Amount of lipid in the cell (mg) | 40 | (81) | 46 | (97) |
| (4) Total amount of lipid (mg) | 102 | (86.2) | 162 | (104) |
| (5) (2)/(4) | 0.61 | (0.06) | 0.72 | (0.07) |
| (6) Amount of γ-linolenic acid in the reaction solution (mg) | 7.9 | (0) | – | |
| (7) Amount of γ-linolenic acid in the cell (mg) | 5.8 | (11.8) | – | |
| (8) Total amount of γ-linolenic acid (mg) | 13.7 | (11.8) | – | |
| (9) Amount of arachidonic acid in the reaction solution (mg) | – | | 3.7 | (0) |
| (10) Amount of arachidonic acid in the cell (mg) | – | | 1.5 | (3.0) |
| (11) Total amount of arachidonic acid (mg) | – | | 5.2 | (3.0) |

EP 0 207 475 B1

Table 2

| Composition of fatty acid | Strain | |
|---|---|---|
| | Mucor ambiguus IFO 6742 | Fusarium oxysporum IFO 5942 |
| Palmitic acid | 13.7 % | 17.5 % |
| Stearic acid | 11.3 % | 10.6 % |
| Oleic acid | 32.4 % | 25.1 % |
| Linoleic acid | 27.2 % | 39.8 % |
| $\gamma$-Linolenic acid | 12.7 % | - |
| Arachidonic acid | - | 3.2 % |
| The others | 2.7 % | 3.8 % |

Table 3

| | Strain | Ratio between amount in the reaction solution and total amount |
|---|---|---|
| 1 | Aspergillus candidus IFO 4309 | 0.63 (0.02) |
| | Penicillium spinulosum IFO 5793 | 0.36 (0.01) |
| | Mucor ambiguus IFO 6742 | 0.48 (0) |
| | Rhizopus oryzae IFO 5418 | 0.58 (0) |
| 2 | Porphyridium cruentum | 0.55 (0) |
| 3 | Rhizopus stolonifer IFO 4781 | 0.65 (0.01) |
| | Absidia coerulea IFO 4011 | 0.35 (0.03) |
| | Actinomucor repens HUT 1049 | 0.54 (0) |
| | Choanephora circinans IFO 5991 | 0.62 (0) |
| | Cunninghamella echinuleta var. elegans IFO 4441 | 0.53 (0.02) |
| | Phycomyces nitens IFO 5694 | 0.55 (0) |
| | Rhizomucor miehei IFO 9740 | 0.58 (0) |
| | Helicostylum nigricans IFO 8091 | 0.46 (0) |
| | Paecilomyces varioti HUT 1229 | 0.48 (0.01) |
| | Syncephalastrum nigricans HUT 1229 | 0.24 (0) |
| | Circinella rigida IFO 6411 | 0.30 (0) |
| | Gongronella butleri IFO 8080 | 0.54 (0) |
| | Backusella circina IFO 9231 | 0.34 (0.02) |
| | Pythium debaryanum IFO 5919 | 0.29 (0) |
| | Fennellomyces linderi IFO 6409 | 0.51 (0) |
| 4 | Spirulina platensis | 0.49 (0) |
| 5 | Chlorella pyrenoidosa | 0.45 (0) |

Example 2

Various fungi were cultured by employing the (A) medium as in Example 1 under the same condition to produce lipid. Table 4 shows the ratio between an amount of the lipid in the reaction soution and a total amount of the lipid, figures in parenthesis showing the results of the control wherein the surfactants was not added.

7

Table 4

| Strain | Ratio between amount of lipid in the reaction solution and total amount of lipid |
|---|---|
| Cladosporium herbarum IFO 6348 | 0.51 (0.08) |
| Trichoderma viride IFO 9066 | 0.26 (0.09) |
| Pellicularia filamentosa IFO 6476 | 0.54 (0.02) |
| Gliocladium virenes IFO 9169 | 0.32 (0.02) |
| Humicola grisea IFO 4868 | 0.52 (0) |
| Claviceps purpurea IFO 5782 | 0.63 (0.07) |
| Cephalosporium chrysogenum IFO 30055 | 0.44 (0.01) |
| Gibberella fujikuroi IFO 9976 | 0.37 (0.04) |
| Tremella mesenterica IFO 9313 | 0.46 (0.02) |

Example 3

Mucor ambiguus IFO 6742 and Fusarium oxysporum IFO 5942 were shake-cultured on the (A) medium or on the (B) medium according to the procedure as in Example 1 to examine the effect of the various surfactants. Table 5 shows the ratio between an amount of the lipid in the reaction solution and a total amount of the lipid for each surfactants together with the control wherein no surfactants was added.

Table 5

| Surfactants | | Ratio between amount of lipid in the reaction solution and total amount of lipid | |
|---|---|---|---|
| | | Mucor ambiquus IFO 6742 | Fusarium oxysporum IFO 5942 |
| Control | | 0.06 | 0.07 |
| Nissan nonion OT-221 | 1.0 % | 0.51 | 0.56 |
| Plonon 201 | 1.0 % | 0.33 | 0.23 |
| Nissan nonion OP-85R | 1.0 % | 0.11 | 0.16 |
| Emanon 4110 | 1.0 % | 0.38 | 0.35 |
| Demol EP | 0.5 % | 0.15 | 0.26 |
| Emal 20C | 0.05 % | 0.35 | 0.30 |
| Sannol NES | 0.5 % | 0.22 | 0.12 |
| Pelex OT-P | 0.01 % | 0.17 | 0.30 |

The composition of fatty acid in the thus obtained lipid was similar to that shown in Table 2 in Example 1, i.e. content of $\gamma$-linolenic acid is 8 to 20 % with respect to Mucor ambiguus IFO 6742 and content of arachidonic acid is 1 to 4 % with respect to Fusarium oxysporum IFO 5942.

Example 4

Mucor ambiguus IFO 6742 and Fusarium oxysporum IFO 5942 were shake-cultured on the (A) medium or on the (B) medium according to the procedure as in Example 1 to prepare the seed cells.

When the microorganism supporting material is employed, about 1 ℓ of the microorganism supporting material, polyurethane in the form of block or nylon in the form of block, each having side length of 6 mm, porosity of 95 to 98 % and pore number of 30 pores/cm, together with the above obtained seed cell were cultured with aeration by means of the conventional bubble tower in 2 ℓ of the same medium as in Example 1. The microorganisms were physically adsorbed on the supporting material after 100 hours of the culture. Successively, the reaction was continued in the presence of surfactants to produce lipid.

When alginic acid was employed as a gelling agent, the seed cells prepared as above were collected by centrifugation and suspended in 2 % solution of sodium alginate. The suspension was dropped through the nozzle to a solution of 0.1 M CaCℓ$_2$, which was previously stirred, to prepare sphere gel having about 4 mm of diameter. When carrageenan was employed as a gelling agent, similarly cells were suspended in a carrageenan solution and the suspension was dropped to a 2 % solution of KCl to prepare sphere gel having about 4 mm of diameter. Bubble tower was charged with about 1 l of the thus obtained sphere gel and the reaction was conducted in the presence of surfactants to produce lipid.

The reaction was carried out employing the same medium as that employed for preparing the seed cells at 30ºC at pH 3 to 5 with a quantity of airflow of 1 vvm while renewing the reaction solution three times at every seven days interval. The surfactants Rheodol TW-0320 was added at the concentration of 1.0 % by weight in the reaction solution.

By measuring an amount of lipid in the reaction solution, the results shown in Table 6 was obtained.

Table 6

| Immobilization | Amount of lipid in the reaction solution | | | | | |
| | Mucor ambiguus IFO 6742 | | | Fusarium oxysporum IFO 5942 | | |
| | 1st | 2nd | 3rd | 1st | 2nd | 3rd |
| Polyurethane in the form of block | 1.5 | 2.0 | 1.9 | 1.5 | 1.3 | 1.8 |
| Nylon in the form of block | 1.2 | 1.7 | 1.8 | 1.7 | 1.5 | 1.2 |
| Sodium alginate gel | 0.5 | 1.4 | 1.6 | 0.7 | 1.2 | 1.4 |
| Carrageenan gel | 0.7 | 1.6 | 1.5 | 0.9 | 1.3 | 1.3 |

Content of γ-linolenic acid or arachidonic acid in the thus obtained lipid was similar to that obtained in Example 1, i.e. content of γ-linolenic acid is 9 to 18 % with respect to Mucor ambiguus IFO 6742 and content of arachidonic acid is 2 to 4 % with respect to Fusarium oxysporum IFO 5942.

Example 5

10

By employing the immobilized microorganism prepared according to the procedure as in Example 4 wherein Mucor ambiguus IFO 6742 was immobilized on polyurethane in the form of block, continuous culture was conducted in the same bubble tower. The fresh medium having the same composition as that of the (A) medium in Example 1 except that content of glucose is 0.4 % was continuously introduced by means of pump at a rate of 200 ml/h while the reaction solution was continuously drained from the part of the bubble tower at the same rate as that of the introduction. The surfactants Rheodol TW-0320 was added at the concentration of 1.0 % by weight in the reaction solution. After stationary state was obtained, the lipid in the effluent was analyzed. As the result, a concentration of lipid in the reaction solution and a concentration of $\gamma$-linolenic acid in the reaction solution were 65 mg/$\ell$ and 8.2 mg/$\ell$, respectively.

## Claims

1. A process for preparing secreted lipids optionally in combination with non-secreted lipids by employing fungi or algae capable of biosynthesizing lipids which comprises conducting the reaction in a reaction system wherein surfactants are present, characterized in that immobilized microorganism particle of fungi or algae is employed.

2. The process of Claim 1, wherein the fungi or algae capable of biosynthesizing lipids is selected from the genus of Aspergillus, Fusarium, Penicillium, Porphyridium, Mucor, Rhizopus, Absidia, Actinomucor, Choanephora, Cunninghamella, Phycomyces, Rhizomucor, Helicostylum, Paecilomyces, Syncephalastrum, Circinella, Gongronella, Backusella, Pythium, Fennellomyces, Spirulina, Cladosporium, Trichoderma, Pellicularia, Gliocladium, Humicola, Claviceps or Chlorella.

3. The process of Claim 1, wherein the surfactants is anionic surfactants or nonionic surfactants.

4. The process of Claim 3, wherein the anionic surfactants is selected from the group consisting of polycarboxylate type high molecular surface active agent, polyoxyethylene alkyl ether sulfate, polyoxyethylene alkyl aryl ether sulfate, dialkyl sulfosuccinate and mixture thereof.

5. The process of Claim 3, wherein the nonionic surfactants is selected from the group consisting of polyoxyethylene sorbitan fatty acid ester, polyoxyethylene polypropylene blockpolymer, sorbitan fatty acid ester, polyethylene glycol fatty acid ester and mixture thereof.

6. The process of Claim 1, wherein the immobilized microorganism particle is obtained by employing a gelling agent or microorganism supporting material.

7. The process of Claim 6, wherein the gelling agent is polyacrylamide, alginic acid, carrageenan, collagen or urethane polymer.

8. The process of Claim 6, wherein the microorganism supporting material is polymer foam material such as polyolefine polymer, diene polymer, polyurethane polymer, vinyl polymer, condensation polymer, silicone or fluorine-contained resin.

9. The process of Claim 6, wherein the microorganism supporting material is porous material or processes metal material having a porosity of 70 to 99 % selected from the group consisting of ceramic, glass, active carbon, pumice and metal.

## Patentansprüche

1. Verfahren zur Herstellung sekretierter Lipide, gegebenenfalls in Kombination mit nichtsekretierten Lipiden, durch Verwendung von Pilzen oder Algen, die zur Biosynthese von Lipiden fähig sind, umfassend die Durchführung einer Reaktion in einem Reaktionssystem in Gegenwart von Surfactanzien, dadurch gekennzeichnet, daß man immobilisierte Mikroorganismuspartikel von Pilzen oder Algen verwendet.

2. Verfahren nach Anspruch 1, worin die zur Biosynthese von Lipiden fähigen Pilze oder Algen ausgewählt sind aus der Gattung Aspergillus, Fusarium, Penicillium, Porphyridium, Mucor, Rhizopus, Absidia, Actinomucor, Choanephora, Cunninghamella, Phycomyces, Rhizomucor, Helicostylum, Paecilomyces, Syncephalastrum, Circinella, Gongronella, Backusella, Pythium, Fennellomyces, Spirulina, Cladosporium, Trichoderma, Pellicularia, Gliocladium, Humicola, Claviceps oder Chlorella.

**3.** Verfahren nach Anspruch 1, worin das Surfactant ein anionisches oder nichtionisches Surfactant ist.

**4.** Verfahren nach Anspruch 3, worin das anionische Surfactant ausgewählt ist aus der Gruppe bestehend aus einem polycarboxylatartigen hochmolekularen oberflächenaktiven Mittel, Polyoxyethylen-Alkylethersulfat, Polyoxyethylen-Alkylarylethersulfat, Dialkylsulfosuccinat und Mischungen derselben.

**5.** Verfahren nach Anspruch 3, worin das nichtionische Surfactant ausgewählt ist aus der Gruppe bestehend aus Polyoxyethylen-Sorbitanfettsäureester, Polyoxyethylen-Polypropylen-Blockpolymer, Sorbitanfettsäureester, Polyethylenglykolfettsäureester und Mischungen derselben.

**6.** Verfahren nach Anspruch 1, worin das immobilisierte Mikroorganismuspartikel durch Anwenden eines Geliermittels oder eines Trägermaterials für Mikroorganismen erhalten wird.

**7.** Verfahren nach Anspruch 6, worin das Geliermittel Polyacrylamid, Alginsäure, Carrageenan, Collagen oder ein Urethanpolymer ist.

**8.** Verfahren nach Anspruch 6, worin das Trägermaterial für den Mikroorganismus aus einem Polymerschaummaterial wie einem Polyolefinpolymer, einem Dienpolymer, einem Polyurethanpolymer, einem Vinylpolymer, einem Kondensationspolymer, Silikon oder einem fluorhaltigen Harz ist.

**9.** Verfahren nach Anspruch 6, worin das Trägermaterial für den Mikroorganismus ein poröses Material oder ein verarbeitetes Metallmaterial einer Porosität von 70 bis 99% ist, ausgewählt aus der Gruppe bestehend aus Keramik, Glas, Aktivkohle, Bimsstein und Metall.

**Revendications**

**1.** Procédé de préparation de lipides sécrétés, éventuellement en combinaison avec des lipides non sécrétés, grâce à l'emploi de moisissures ou d'algues capables de biosynthétiser des lipides, qui comprend la réalisation de la réaction dans un système réactionnel dans lequel sont présents des tensioactifs, caractérisé en ce que l'on emploie une particule de microorganisme immobilisée de moisissures ou d'algues.

**2.** Procédé selon la revendication 1, dans lequel les moisissures ou les algues capables de biosynthétiser des lipides sont choisies parmi les genres Aspergillus, Fusarium, Penicillium, Porphyridium, Mucor, Rhizopus, Absidia, Actinomucor, Choanephora, Cunninghamella, Phycomyces, Rhizomucor, Helicostylum, Parcilomyces, Syncephalastrum, Circinella, Gongronella, Backusella, Pythium, Fennellomyces, Spirulina, Cladosporium, Trichoderma, Pellicularia, Gliocladium, Humidola, Claviceps ou Chlorella.

**3.** Procédé selon la revendication 1, dans lequel les tensioactifs sont des tensioactifs anioniques ou des tensioactifs non ioniques.

**4.** Procédé selon la revendication 3, dans lequel les tensioactifs anioniques sont choisis dans le groupe constitué par un agent tensioactif de masse moléculaire élevée, de type polycarboxylate, un alkyléthersulfate polyéthoxylé, un alkylaryléthersulfate polyéthoxylé, un dialkylsulfosuccinate et un mélange de ceux-ci.

**5.** Procédé selon la revendication 3, dans lequel les tensioactifs non ioniques sont choisis dans le groupe constitué par un ester d'acide gras et de sorbitanne polyéthoxylé, un polymère séquencé polyoxyéthylène-polyoxypropylène, un ester d'acide gras et de sorbitanne, un ester d'acide gras et de polyéthylèneglycol et un mélange de ceux-ci.

**6.** Procédé selon la revendication 1, dans lequel la particule de microorganisme immobilisée s'obtient grâce à l'emploi d'un agent gélifiant ou d'un support de microorganisme.

**7.** Procédé selon la revendication 6, dans lequel l'agent gélifiant est un polyacrylamide, l'acide alginique, la carragénine, le collagène ou un polymère d'uréthanne.

8. Procédé selon la revendication 6, dans lequel le support de microorganisme est une mousse polymère telle qu'un polymère polyoléfinique, un polymère diénique, un polymère polyuréthanne, un polymère vinylique, un polymère de condensation, une silicone ou une résine fluorée.

9. Procédé selon la revendication 6, dans lequel le support de microorganisme est une matière poreuse ou une matière métallique industrielle ayant une porosité de 70 à 99%, choisie dans le groupe constitué par la céramique, le verre, le charbon actif, la pierre ponce et un métal.